Europäisches Patentamt

European Patent Office

Office européen des brevets

(19)

(11) Publication number: **0 263 552 B1**

# EUROPEAN PATENT SPECIFICATION

(12)

(45) Date of publication of patent specification: **18.03.92**

(51) Int. Cl.⁵: **B32B 27/36**, A61L 15/07, C08L 67/04, C08L 75/04

(21) Application number: **87201839.5**

(22) Date of filing: **02.10.87**

(54) **Composite material for medical or paramedical, particularly orthopaedic use and method for manufacturing it.**

(30) Priority: **08.10.86 BE 2061067**
**21.11.86 BE 2061090**
**19.12.86 BE 2061130**

(43) Date of publication of application:
**13.04.88 Bulletin 88/15**

(45) Publication of the grant of the patent:
**18.03.92 Bulletin 92/12**

(84) Designated Contracting States:
**AT BE CH DE ES FR GB GR IT LI LU NL SE**

(56) References cited:
**EP-A- 0 087 329**
**US-A- 3 728 206**

(73) Proprietor: **Ponnet, Tom Paul Marthe Ghislain**
**Statiestraat 92**
**B-2600 Berchem(BE)**

(72) Inventor: **Ponnet, Tom Paul Marthe Ghislain**
**Statiestraat 92**
**B-2600 Berchem(BE)**

(74) Representative: **Debrabandere, René et al**
**BUREAU DE RYCKER Vereenigde Octrooibureaux Belgie N.V. Arenbergstraat 13**
**B-2000 Antwerpen(BE)**

Rank Xerox (UK) Business Services

EP 0 263 552 B1

## Description

The invention relates to a composite material for medical or paramedical, particularly orthopaedic use, which comprises a thermoplastic composition based on polycaprolactone and polyurethane.

A thermoplastic composition based on polycaprolactone and polyurethane is known from EP-A- 0 087 329.

The polyurethane is formed in situ in soft form in the polycaprolactone from a polyol and at least one poly-isocyanate.

The resulting polycaprolactone-polyurethane composition is manufactured in the shape of plates or sheets, and it is being used as such to replace plaster bandages.

The composition is made soft and self-adhesive by heating same above 60° C, by dipping into hot water, and thereafter is put in the desired shape on the body. Parts of the material stick to one another. After cooling, the material forms a rigid unit.

The arrangement in position of such material is however not easy because it is highly self-adhesive in plastic condition. Once portions thereof have been brought against one another, it is substantially impossible to release these portions thereafter. Correcting faults when applying the material is then also impossible in practice. Moreover, the material cools very fast, in such a way that it also becomes rigid again quite fast and the time interval during which said material can be applied in position is limited.

The composite material is also in direct contact with the body. This may not only cause allergic reactions due to chemicals possibly still present in the material, but it is mostly uncomfortable due to the relatively high temperature at the beginning of the application. Due to direct contact of the material with the skin, breathing through the skin is hampered.

There is known from US-A-3,728,206, a material based on polyurethane and polycaprolactone, but such material is formed by impregnating non-thermoplastic polyurethane foam with a caprolactone for example.

Said material which is also used for replacing plaster bandages is softened by heating above 49° C,preferably above 82° C.

It is however so strongly adhesive that it is generally protected with a coating, for example an aluminum foil. When said material is handled in rolls, it is coated on one side with silicone-processed paper to prevent the windings sticking to one another.

The requirement of having to remove such protection when using the material makes the application thereof difficult.

Moreover with this material also, no correction is possible any more once portions of the material in plastic condition have contacted one another. Releasing such portions is substantially impossible.

Direct contact of the material with the skin should further be avoided, on the one hand possibly because of the relatively high temperature which is required to make the material plastic and workable, and on the other hand to prevent the action of possible chemicals on the skin. This is the reason why according to the US patent, the material is not laid directly on the body, but an orthopaedic stocking is first put on. Thereby the drawbacks of the direct contact are indeed avoided and the skin can breathe, but the orthopaedic stocking is relatively costly and the requirement of such a stocking makes the application of the orthopaedic bandage time-consuming and more difficult.

The invention has for object to obviate said drawbacks and to provide a composite material for medical or paramedical, particularly orthopaedic use, the use of which is very simple and unexpensive and notably plastic portions of which brought against one another, may be released again, which composite material can be applied directly on the skin without danger or uncomfortable feeling and lets the skin breathe, in such a way that the use of orthopaedic stockings or similar protections between the skin and the material is superfluous.

For this purpose the layer of the thermoplastic composition is provided on at least one side with a coating of foam plastic material.

The composite material is naturally laid with the foam plastic coating against the skin. The plastic foam forms an insulating layer which avoids the hot thermoplastic composition contacting directly the skin, delays the composition cooling and lets the skin breathe. The foam plastic coating, before and during the application, is in no way filled with thermoplastic material as for example with the foam plastic from the composite material according to US-A-3,728,206, which foam plastic does not form a coating.

According to the invention, the foam plastic forms a coating which prevents in no way portions of the composite material sticking to one another. On the one hand, by exerting a small pressure, portions of the thermoplastic composition may still stick to one another through the foam plastic coating, as the composition squeezes through the plastic foam, but on the other hand due to the presence of the plastic foam, such portions sticking to one another may still be released as long as the composition is still in soft condition.

In a preferred embodiment of the invention, the layer of thermoplastic composition is coated on both sides with a foam plastic coating and the material comprises consequently a core of the thermoplastic composition and on both sides thereof, a foam plastic coating.

In this embodiment, the material may be laid with whatever side against the skin. Gripping the material with bare hands can be done, as due to the foam plastic coating, the excessive sticking is avoided. As the material core is lined on either side with plastic foam, cooling of said core is strongly slowed-down and the material can be shaped for a longer time.

In an advantageous embodiment of the invention, the foam plastic coating has a thickness between 0.05 and 1.5 mm.

Mostly with this embodiment, there is obtained the desired adhesive power for portions of the composite material which are brought against one another through the foam plastic coating. As long as the thermoplastic composition can still be shaped, the adhesive power is limited enough to let such portions still be released from one another. After some time and certainly after the thermoplastic material has set, the portions adhere very strongly to one another.

In an advantageous embodiment of the invention, the foam plastic coating is provided with perforations.

In another advantageous embodiment of the invention, the composite material is provided with perforations cross-wise through the core and coating.

In a useful embodiment of the invention, the thermoplastic composition comprises 20 to 70 weight-% polyurethane and 80 to 30 weight-% polycaprolactone.

In a particularly useful embodiment of the invention, the polyurethane in the thermoplastic composition is a caprolactone polyester polyurethane.

In a remarkable embodiment of the invention, the layer of the thermoplastic composition is manufactured starting from a mixture of a polycaprolactone granulate and a polyurethane granulate.

As opposed to the teaching in EP-A-0 087 329, it appears that according to the invention, the polyurethane does not necessarily have to be formed in soft condition in the polycaprolactone.

When one starts with granulates of polycaprolactone and polyurethane, there is obtained according to the invention a composite material which still has sufficient mechanical properties.

The invention further pertains to a method which is particularly suitable for manufacturing a composite material according to one of the above embodiments.

The invention thus also pertains to a method for manufacturing a composite material for medical or paramedical, particularly orthopaedic use, based on polycaprolactone and polyurethane, which method has as characteristic that one mixes and regranulates a polycaprolactone granulate and a polyurethan granulate, one brings the resulting granulate under the action of pressure or heat, or both in layer form, and one coats the resulting layer over at least one side thereof with a foam plastic layer.

Other features and advantages of the invention will stand out from the following description of a composite material for medical or paramedical, particularly orthopaedic use, and of a method for manufacturing such a composite material, according to the invention; said description is only given by way of example and does not limit the invention; the reference numerals pertain to the accompanying drawings.

Figure 1 shows a cross-section of part of a composite material according to the invention.

Figure 2 shows a block diagram of a method for manufacturing said composite material.

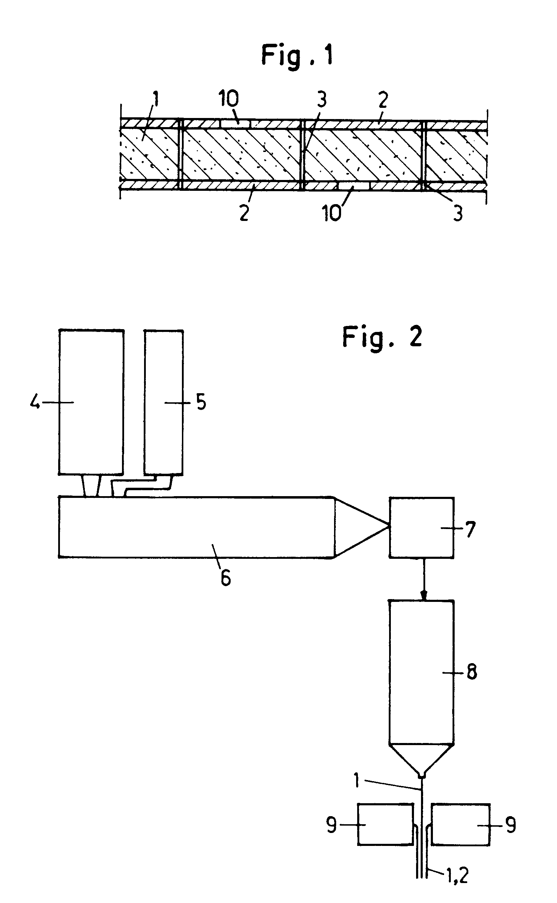

The composite material as shown in figure 1 is comprised of a core 1 from a thermoplastic composition comprised of 20 to 70 weight-% and preferably 50 to 60, for example 55 weight-% polyurethane,and 80 to 30 weight-% and preferably 50 to 40 weight-%, for example 45 weight-% polycaprolactone, and on either side of said core, a foam plastic coating 2.

The molecular weight of the polyurethan preferably lies between 10,000 and 100,000, and the molecular weight of the polycaprolactone preferably lies between 10,000 and 60,000,in particular preferably between 37,000 and 50,000.

Polyester polyurethanes are mostly suitable as polyurethane.

Caprolactone polyester polyurethan is particularly suitable, which polyurethane may be obtained by reacting isocyanate and polycaprolactone-based polyester.

Such a caprolactone polyester polyurethane is put on the market as a granulate by B.F.Goodrich Belgium under the name Gamma Estane[R], type 5720.

The melting point of said polycaprolactone polyester polyurethane lies between 190 and 210°C. By adding the polycaprolactone, also preferably in granulate form, there is obtained a thermoplastic composition which is already distortable and kneadable by a temperature of 69°C, and remains distortable by cooling down to about 50°C.

At this temperature, the core 1 of said material can be stretched up to four times the original length thereof.

In cold condition, the thermoplastic composition is relatively rigid.

The thickness of core 1 normally lies between 0.5 and 10 mm.

The coatings 2 to the contrary are markedly thinner. They have a thickness between 0.05 and 1.5 mm that preferably amounts to about 0.6 mm and are preferably from so-called soft plastic foam.

Suitable foam plastics for the coatings 2 are polyurethane, particularly polyester polyurethane, and polyether foam.

For some applications, one or both coatings 2 may be provided with perforations 10 with a diameter of at least 0.5 mm and for example 2 mm. Such perforations are for example required when heating the composite material occurs in a warm water bath. The plastic foam absorbes water. Even after squeezing the water out of the plastic foam, there still remains a water fraction which opposes sticking. In the location of part at least of the perforations there is no water, and the plastic from core 1 of one layer can contact the plastic from the core 1 of an above-lying layer.

Cross-wise through core 1 and coatings 2, generally smaller perforations 3 with a diameter of at least 0.5 mm may be provided, so as not to hamper the skin breathing after applying the material.

As shown in figure 2, the composite material may be manufactured by feeding a polycaprolactone granulate from a supply bin 4 and a polyurethane granulate from a supply bin 5, to a twin screw extruder. During the extrusion, the granulates are mixed and under the action of pressure and possibly heat, they become fluid. A string of the polyurethane-polycaprolactone composition is pressed out of extruder 6. Said string is fed to the chopper 7 where it is chopped into small pieces. In this way, new granulates are obtained from the thermoplastic composition of polycaprolactone and polyurethane.

Immediately thereafter or some time later, said granulates are again fed to a second extruding device 8, whereby thus the thermoplastic composition is extruded in the form of a layer which comprises the core 1.

By means of foam sprayers 9, a coating 2 of foam plastic is finally sprayed over both sides of core 1.

It is naturally also possible to manufacture the coatings 2 separately in the form of foam plastic layers and thereafter to apply same on the adhesive layer which comes out of extruding device 8.

Said last way is appropriate when the coatings 2 only are provided with perforations 10. The perforations may be made in the coatings 2 before said coatings are applied on core 1. When the perforations 3 have to extend through the coatings 2 and core 1, said perforations 3 may naturally be made after applying the coatings 2 on core 1.

Due to the presence of coatings 2 of plastic foam, the composite material may be applied directly in distortable condition on the skin. It does not stick to hair and skin. It is soft to the touch. It does not either leave visible fingerprints, so that throw-away gloves do not have to be used for the application. It does let X-rays well through.

Portions of the composite material 1, 2 which are brought one against the other, for example the ends of a strip of such material, adhere in the plastic condition of core 1, sufficiently to one another to remain sticking to one another, but not so strongly that they can not be released any more from one another. The core portions always adhere through two thin layers of coating 2. After some time however, under the action of the pressure from the above-lying material portion, the distortable plastic squeezes through the thin layers of coating 2 and after cooling, the portions strongly stick to one another. After being heated again, the portions may however be pulled away again from one another.

When applying the composite material, corrections may thus easily be made and a piece from the composite material may also be used anew a plurality of times.

Possible residues from the material are also not lost. A plurality of material layers may be laid over one another in plasticized condition.

As the coatings 2 form a thin insulating layer, the working time in the plasticized condition of core 1, is relatively long. Moreover, the skin is subjected to little trouble from the relatively high temperature which is required to make the core 1 plastic and distortable.

The skin may still breathe even after application of the material.

Heating the material to plasticize the core preferably occurs in dry condition, for example by means of hot air, although heating in a liquid is possible. In this latter case, the liquid has to be pressed out of the plastic coating before making use of the composite material.

The composite material is relatively homogeneous, does not crumble and is simple to work with, wear-resistant, withstands chemicals and ageing processes, and is impact-resistant.

The composite material is thus particularly suitable for replacing plaster bandages, splints or similar.

The invention will be further explained with reference to the following examples.

Example 1

A mixture from 50 weight-% polycaprolactone and 50 weight-% caprolactone polyester polyurethane obtained by reacting isocyanate and a polycaprolactone-based polyester, in the shape of granulates, is regranulated and extruded in the form of a strip with a thickness of 1.4 mm maximum and 0.5 mm minimum. Said strip is coated on both sides thereof with a coating 2 of polyester polyurethane foam with 0.6 mm thickness, which is provided with perforations 10 with a diameter of at least 0.5 mm, which take up a total surface area of 40% at the most of the coating.

After such shaping, the strip of composite material is further provided with additional perforations 3 which are made cross-wise through the core 1 and coatings 2 to avoid hampering of the breathing of the skin pores after application thereof.

The strip in still plastic condition is rolled-up and cooled.

To be used, the strip and thus more particularly the core 1 is plasticized again by dipping the strip in a water bath of about 72°C.

The strip roll is taken out of the bath, it is left to drip for a few seconds, whereby the strip cools down to about 50°C and the excess water is pressed out of the roll. The required amount of strip is unwound from the roll and it is laid to replace a plaster bandage , around a part of the body.

Example 2

The composite material is manufactured in the same way as in example 1, but inside the second extruder, the core 1 from the thermoplastic composition is extruded in the shape of a plate with a thickness between 1 and 10 mm.

The core 1 is coated on either side with a coating 2 of unperforated polyester polyurethane foam with 0.6 mm thickness.

The resulting composite material in the form of a plate is particularly suitable to be used as splint which is brought to the required shape in soft condition.

To plasticize this composite material, it is heated to approximately 80°C during about 5 minutes inside an oven, on a hot plate or with hot air.

Shortly after heating is stopped, the temperature of core 1 drops down to about 60°C, whereafter the temperature drops quite slowly. Due to the plastic, the composite material even with this temperature of 60°C of the core, may be applied without any problem to the skin. The composite material provides a comfortable warmth and may be laid even on the most tender parts of the body.

The above-described composite material may be used in a lot of applications. The arrangement or applying thereof is quite easy. Portions from the material simply put on one another in plastic condition, do not stick or very lightly to one another, and it is but when such portions are pressed together that a fastening is obtained which, after becoming rigid, is very strong. As long as such cooling has not occured, the portions may still be loosened from one another.

The composite material is also very temperature resistant. Even should the material be excessively heated, for example to 150°C during thirty minutes, which is not excluded as heating occurs preferably not in a water bath but inside an oven, on a hot plate or in a hot air stream, the composite material does not lose in any way the properties thereof. It is only necessary to wait somewhat longer before the composite material can be used.

The coatings 2 from foam plastic also insure anti-slip properties. Once a bandage or splint from said composite material has been laid on the body, the foam plastic opposes the sliding of the bandage or splint. This is mostly of importance when for example, the material is arranged on moving limbs such as a foot for example.

The invention is in no way limited to the above-described embodiments and within the scope of the described embodiments, many changes may be brought notably as regards the composition, the imparted shapes, the size and the applications.

**Claims**

1. Composite material for medical or paramedical, particularly orthopaedic use, which comprises a layer (1) of a thermoplastic composition based on polycaprolactone and polyurethane, characterized in that the layer (1) thermoplastic composition is provided on at least one side with a coating (2) from foam plastic material.

2. Composite material according to claim 1, characterized in that the layer (1) of thermoplastic composition is coated on both sides with a foam plastic coating (2) and the material comprises consequently a core (1) of the thermoplastic composition and on both sides thereof, a foam plastic coating (2).

3. Composite material according to any one of claims 1 and 2, characterised in that the the foam plastic coating (2) has a thickness between 0.05 and 1.5 mm.

4. Composite material according to claim 3, characterized in that the foam plastic coating (2) has a thickness of about 0.60 mm.

5. Composite material according to any one of claims 1 to 4, characterized in that the plastic foam of the coating (2), is a polyurethane foam.

6. Composite material according to any one of claims 1 to 5, characterized in that the foam plastic coating (2) is provided with perforations (10).

7. Composite material according to any one of claims 1 to 6, characterized in that it is provided with perforations (3) cross-wise through the core (1) and coating (2).

8. Composite material according to either one of claims 6 and 7, characterized in that the perforations (3 or 10) have a diameter of at least 0.5 mm.

9. Composite material according to any one of claims 1 to 8, characterized in that the thermoplastic composition contains 20 to 70 weight-% polyurethane, and 80 to 30 weight-% polycaprolactone.

10. Composite material according to claim 9, characterized in that the thermoplastic composition contains 50 to 60 weight-% polyurethane.

11. Composite material according to any one of claims 1 to 10, characterized in that the polyurethan from the thermoplastic composition is a caprolactone polyester polyurethane.

12. Composite material according to any one of claims 1 to 11, characterized in that the thermoplastic composition is comprised of polycaprolactone with a molecular weight of 10,000 to 60,000, and polyurethane with a molecular weight of 10,000 to 100,000.

13. Composite material according to any one of claims 1 to 12, characterized in that the layer (1) of thermoplastic composition is manufactured starting from a mixture of a polycaprolactone granulate and a polyurethane granulate.

14. Method for manufacturing a composite material for medical or paramedical, particularly orthopaedic use, based on polycaprolactone and polyurethane, characterized in that one mixes and regranulates a polycaprolactone granulate and a polyurethane granulate, one brings the resulting granulate under the action of pressure or heat, or both in the form of a layer (1), and one coats the resulting layer (1) on at least one side thereof with a foam plastic layer (2).

15. Method according to claim 14, characterized in that the polycaprolactone and polyurethane granulates are extruded, the resulting product is chopped into small pieces, and said small pieces are extruded again to form a layer (1) which is coated finally on at least one side with a layer of (2) plastic foam.

**Revendications**

1. Matière composite destinée à une utilisation médicale ou paramédicale, en particulier à une utilisation orthopédique, qui comprend une couche (1) d'une composition thermoplastique à base de polycaprolactone et de polyuréthanne, **caractérisée en ce que** la couche (1) de la composition thermoplastique est munie, sur au moins un côté, d'un revêtement (2) en mousse de matière plastique.

2. Matière composite selon la revendication 1, **caractérisée en ce que** la couche 1 de composition thermoplastique est recouverte des deux côtés à l'aide d'un revêtement (2) en mousse de matière plastique et la matière comprend, en conséquence, une partie centrale (1) de composition thermoplastique et, sur les deux côtés de celle-là, un revêtement (2) en mousse de matière plastique.

3. Matière composite selon l'une quelconque des revendications 1 ou 2, **caractérisée en ce que** le revêtement (2) en mousse de matière plastique a une épaisseur entre 0,05 et 1,5 mm.

4. Matière composite selon la revendication 3, **caractérisée en ce que** le revêtement (2) en mousse de matière plastique a une épaisseur d'environ 0,60 mm.

5. Matière composite selon l'une quelconque des revendications 1 à 4, **caractérisée en ce que** la mousse plastique du revêtement (2) est de la mousse de polyuréthanne.

6. Matière composite selon l'une quelconque des revendications 1 à 5, **caractérisée en ce que** le revêtement (2) en mousse de matière plastique est muni de perforations (10).

7. Matière composite selon l'une quelconque des revendications 1 à 6, **caractérisée en ce qu**'elle est munie de perforations (3) transversalement à travers la partie centrale (1) et le revêtement (2).

8. Matière composite selon l'une quelconque des revendications 6 ou 7, **caractérisée en ce que** les perforations (3 ou 10) ont un diamètre d'au moins 0,5 mm.

9. Matière composite selon l'une quelconque des revendications 1 à 8, **caractérisée en ce que** la composition thermoplastique contient de 20 à 70 pour cent en poids de polyuréthanne et de 80 à 30 pour cent en poids de polycaprolactone.

10. Matière composite selon la revendication 9, **caractérisée en ce que** la composition thermoplastique contient de 50 à 60 pour cent en poids de polyuréthanne.

11. Matière composite selon l'une quelconque des revendications 1 à 10, **caractérisée en ce que** le polyuréthanne de la composition thermoplastique est un polyester-polyuréthanne de caprolactone.

12. Matière composite selon l'une quelconque des revendications 1 à 11, **caractérisée en ce que** la composition thermoplastique comprend du polycaprolactone ayant un poids moléculaire de 10.000 à 60.000 et du polyuréthanne ayant un poids moléculaire de 10.000 à 100.000.

13. Matière composite selon l'une quelconque des revendications 1 à 12, **caractérisée en ce qu'**on prépare la couche (1) de composition thermoplastique en partant d'un mélange d'un granulé de polycaprolactone et d'un granulé de polyuréthanne.

14. Procédé pour la préparation d'une matière composite destinée à une utilisation médicale ou paramédicale, en particulier à une utilisation orthopédique, à base de polycaprolactone et de polyuréthanne, **caractérisé** en ce qu'on mélange et on regranule un granulé de polycaprolactone et un granulé de polyuréthanne, on amène le granulé résultant sous l'action de la pression ou de la chaleur ou encore des deux, sous la forme d'une couche (1) et on recouvre la couche résultante (1) sur au moins un de ses côtés à l'aide d'une couche (2) de plastique alvéolaire.

15. Procédé selon la revendication 14, **caractérisé en ce qu'**on extrude les granulés de polycaprolactone et de polyuréthanne, on broie le produit résultant en petits morceaux et on soumet lesdits petits morceaux à une nouvelle extrusion pour obtenir une couche (1) que l'on recouvre finalement, sur au moins un côté, d'une couche (2) de mousse plastique.

**Patentansprüche**

1. Kompositmaterial für medische oder paramedische, besonders orthopädische Verwendung, bestehend aus einer Schicht (1) thermoplastischer Zusammensetzung basierend auf Polycaprolacton und Polyurethan, dadurch gekennzeichnet daß die Schicht (1) thermoplastisches Kompositmaterial an mindestens einer Seite versehen wird von einem Uberzug (2) aus Schaumstoff.

2. Kompositmaterial nach Anspruch 1, dadurch gekennzeichnet daß die Schicht (1) des thermoplastischen Kompositmaterials auf beiden Seiten überzogen wird mit einer Schaumstoffschicht (2) und das Material demzufolge einen Kern (1) des thermoplastischen Kompositmaterials aufweist mit an dessen beiden Seiten einen Schaumstoffüberzug.

3. Kompositmaterial nach einem der Ansprüche 1 und 2, dadurch gekennzeichnet daß der Schaumstoffüberzug (2) eine Starke zwischen 0,05 und 1,5 mm hat.

4. Kompositmaterial nach Anspruch 3, dadurch gekennzeichnet daß der Schaumstoffüberzug (2) eine Stärke hat von etwa 0,6 mm.

5. Kompositmaterial nach einem beliebigen der Ansprüche 1 bis 4, dadurch gekennzeichnet daß der Schaumstoffüberzug (2) ein Polyurethanschaumstoff ist.

6. Kompositmaterial nach einem beliebigen der Ansprüche 1 bis 5, dadurch gekennzeichnet daß der Schaumstoffüberzug (2) versehen ist mit Durchlöcherungen (10).

7. Kompositmaterial nach einem beliebigen der Ansprüche 1 bis 6, dadurch gekennzeichnet daß es versehen ist mit Durchlöcherungen (3) quer durch den Kern (1) und den Uberzug (2).

8. Kompositmaterial nach einem beliebigen der Ansprüche 6 und 7, dadurch gekennzeichnet daß die Durchlöcherungen (3 oder 10) einen Durchmesser von mindesten 0,5 mm haben.

9. Kompositmaterial nach einem beliebigen der Ansprüche 1 bis 8, dadurch gekennzeichnet daß die thermoplastische Zusammensetzung 20 bis 70 Gewichtsprozente Polyurethan enthält und 80 bis 30 Gewichtsprozente Polycaprolacton.

10. Kompositmaterial nach Anspruch 9, dadurch gekennzeichnet daß die thermoplastische Zusammensetzung 50 bis 60 Gewichtsprozente Polyurethan enthält.

11. Kompositmaterial nach einem beliebigen der Ansprüche 1 bis 10, dadurch gekennzeichnet daß das Polyurethan der thermoplastischen Zusammensetzung Caprolacton Polyester Polyurethan ist.

12. Kompositmaterial nach einem beliebigen der Ansprüche 1 bis 11, dadurch gekennzeichnet daß die thermoplastische Zusammensetzung besteht aus Polycaprolacton mit einem Molekulargewicht von 10.000 bis 60.00 und Polyurethan mit einem Molekulargewicht von 10.000 bis 100.000.

13. Kompositmaterial nach einem beliebigen der Ansprüche 1 bis 12, dadurch gekennzeichnet daß die Schicht (1) aus thermoplastische Zusammensetzung hergestellt worden ist ausgehend von einem Gemisch von Polycaprolactongranulat und einem Polyurethangranulat.

14. Methode zur Herstellung eines Kompositmaterials für medische oder paramedische, besonders orthopädische Verwendung, basierend auf Polycaprolacton und Polyurethan, gekennzeichnet dadurch daß man ein Polycaprolactongranulat und ein Polyurethangranulat mischt und wieder granuliert, das resultierende Granulat wird mittels Druck und Hitze, oder beide, in die Form einer Schicht (1) versetzt und an mindestens einer Seite mit einem Schaumstoffüberzug belegt (2).

15. Verfahren nach Anspruch 14, dadurch gekennzeichnet daß die Polycaprolacton- und Polyurethangranulate extrudiert werden, das resultierende Produkt wird in kleine Teile zerkleinert und wieder extrudiert um eine Schicht (1) zu bilden, die letzten Endes mit einem Schaumstoffüberzug (2) bedeckt wird.

# Fig . 1

# Fig. 2